# FASCICULE DE BREVET EUROPEEN

(11) **EP 1 163 523 B1**
(45) Date de publication et mention de la délivrance du brevet: **19.05.2004**
(21) Numéro de dépôt: 00912731.7
(22) Date de dépôt: 22.03.2000
(51) Int. Cl.: G01N 33/569

(54) **METHODE DE DETERMINATION D'UNE PREDISPOSITION A UNE MALADIE THROMBOEMBOLIQUE VEINEUSE**
VERFAHREN ZUM NACHWEIS EINER PRÄDISPOSITION FÜR VENÖSE THROMBOEMBOLISCHE ERKRANKUNGEN
METHOD FOR DETECTING PREDISPOSITION TO A VENOUS THROMBOEMBOLIC DISEASE

(30) Priorité: 23.03.1999 FR 9903613
(43) Date de publication de la demande: 19.12.2001
(73) Titulaire: INSTITUT NATIONAL DE LA SANTE ET DE LA RECHERCHE MEDICALE (INSERM), 75013 Paris (FR); ASSISTANCE PUBLIQUE - HOPITAUX DE PARIS, 75001 Paris (FR)
(72) Inventeur: EMMERICH, Joseph, F-75001 Paris (FR); FIESSINGER, Jean-No[l, F-75007 Paris (FR); AIACH, Martine, F-92310 Sèvres (FR)
(74) Mandataire: Bernasconi, Jean Raymond
(86) Numéro de dépôt international: PCT/FR2000/000725
(87) Numéro de publication internationale: WO 2000/057186

(56) Documents cités:
- WO-A-90/00061
- WO-A-98/06408
- WO-A-98/17280
- US-A- 5 424 187
- DATABASE BIOSIS [en ligne] BIOSCIENCES INFORMATION SERVICE, PHILADELPHIA, PA, US; FRYER, RICHARD H. ET AL: "Chlamydia species infect human vascular endothelial cells and induced procoagulant activity." retrieved from STN XP002124198 & JOURNAL OF INVESTIGATIVE MEDICINE, (1997) VOL. 45, NO. 4, PP. 168-174.,
- DATABASE MEDLINE [en ligne] US NATIONAL LIBRARY OF MEDICINE (NLM), BETHESDA, MD, US; VISSEREN F L ET AL: "[Atherosclerosis as an infectious disease]. Atherosclerose als infectieziekte." retrieved from STN Database accession no. 1999237434 XP002124199 & NEDERLANDS TIJDSCHRIFT VOOR GENEESKUNDE, (1999 FEB 6) 143 (6) 291-5. REF: 36,
- DATABASE BIOSIS [en ligne] BIOSCIENCES INFORMATION SERVICE, PHILADELPHIA, PA, US; MATTILA, K. J. (1) ET AL: "Role of infection as a risk factor for atherosclerosis, myocardial infarction, and stroke." retrieved from STN XP002124200 & CLINICAL INFECTIOUS DISEASES, (MARCH, 1998) VOL. 26, NO. 3, PP. 719-734.,

## Description

La présente invention est basée sur la mise en évidence d'un lien étroit entre une infection par une bactérie du genre Chlamydia et la maladie thromboembolique veineuse.

L'invention concerne plus particulièrement une méthode de détermination *in vitro* d'une prédisposition à une maladie thromboembolique veineuse chez un sujet dans laquelle on détermine si le sujet a été infecté par une bactérie du genre Chlamydia, et sur l'utilisation d'agents actifs sur les bactéries du genre Chlamydia pour la prévention et/ou le traitement d'une maladie thromboembolique veineuse.

La maladie thromboembolique veineuse est un désordre multifactoriel avec des facteurs de risque à la fois génétiques et acquis (Rosendaal et al, 1997). Les facteurs de risque classiques pour la thrombose veineuse sont associés à une altération des vaisseaux ou une stase (ralentissement, voire arrêt de la circulation sanguine veineuse), dus notamment à un traumatisme, une opération chirurgicale ou une immobilisation. Globalement une prédisposition génétique à une thrombose veineuse n'explique que 40% des cas. Des déficits en antithrombine, en protéine C et en protéine S expliquent notamment de 5 à 10% des cas de maladies thromboemboliques veineuses. Après avoir pris en compte des facteurs de risques acquis conventionnels et des prédispositions génétiques, au moins un tiers des épisodes thrombotiques veineux restent inexpliqués.

Actuellement le traitement le plus couramment utilisé dans des cas de thromboses veineuses est un traitement à base d'anticoagulants. Cependant un tel traitement ne peut être envisagé que pour une durée limitée, en raison d'effets secondaires dangereux. Une administration prolongée d'anticoagulants augmente en effet les risques d'hémorragies, en particulier d'hémorragies cérébrales. Un traitement alternatif est par conséquent recherché.

Le lien entre une infection par *Chlamydia pneumoniae* et l'athérosclérose est bien documenté (Fryer Richard H. et al. Journal of Investigative Medicine (1997). vol. 45 NO. 4 pp. 168-174). L'utilisation d'antibiotiques dans le traitement des maladies cardiovasculaires telles que les infarctus du myocarde ou les maladies des artères coronaires a également été envisagée (WO 90/00061, WO 98/17280, WO 98/06408, Gibbs et al, 1998). Les maladies cardiovasculaires visées sont cependant des maladies du système artériel.

En revanche jusqu'à présent aucune donnée ne suggérait la possibilité d'un lien entre *Chlamydia pneumoniae* et les pathologies du système veineux. Alors qu'un article d'Ong et al, publié en 1996 rapportait la détection par PCR (« polymerase chain reaction ») de *Chlamydia pneumoniae* dans une veine iliaque sur deux sujets contrôles *a priori* exempts de pathologies vasculaires, un article ultérieur (Bartels et al, 1998) rapportait au contraire l'absence de cette bactérie dans les veines saphènes natives de patients ayant subi un pontage coronarien.

Les auteurs de la présente invention ont découvert de manière surprenante un lien étroit entre une infection par une bactérie du genre Chlamydia et une maladie thromboembolique veineuse.

Plus particulièrement les auteurs de l'invention ont montré que des taux élevés en anticorps anti- *Chlamydia pneumoniae* représentent un facteur de risque pour une maladie thromboembolique veineuse.

Sans se lier en aucune manière à un mécanisme d'action précis, les auteurs émettent l'hypothèse qu'une infection chronique des parois des veines par *Chlamydia pneumoniae* pourrait rendre les cellules endothéliales veineuses thrombogènes.

La présente invention a donc pour objet une méthode de détermination *in vitro* d'une prédisposition à une maladie thromboembolique veineuse chez un sujet dans laquelle on détermine si le sujet a été infecté par une bactérie du genre Chlamydia, plus particulièrement *Chlamydia pneumoniae*.

On détermine si le sujet a été infecté par une bactérie du genre Chlamydia par analyse d'un échantillon biologique. Il peut s'agir notamment d'un échantillon de sang, d'urine, de liquide pleural, d'un échantillon obtenu par bronchoscopie ou par lavement bronchoalvéolaire, ou d'un échantillon obtenu par une biopsie, par exemple de l'endothélium vasculaire. On peut alors déterminer si cet échantillon contient des anticorps anti-*Chlamydia* ou s'il contient des bactéries du genre Chlamydia ou des fragments de celles-ci. On peut, par exemple, rechercher la présence d'un composant chlamydial, tel que des liposaccharides ou des protéines membranaires, ou des substances produites par *Chlamydia,* tels que des exopolysaccharides, ou encore des substances produites par les cellules hôtes par induction de *Chlamydia*.

De manière préférentielle, on détermine si le sujet a été infecté par une bactérie du genre Chlamydia en dosant le taux d'anticorps anti-*Chlamydia* dans un échantillon biologique d'un sujet à tester, par exemple un échantillon de sang.

Les titres d'anticorps anti-*Chlamydia* obtenus chez les sujets à tester sont ensuite comparés aux titres d'anticorps obtenus chez des sujets contrôles. Le titre est défini par la dilution maximale de l'échantillon biologique pour laquelle les anticorps sont encore détectés et est exprimé par l'inverse du facteur de dilution. Un titre d'anticorps supérieur à 256 peut être considéré comme représentant un facteur de risque non négligeable.

La présente invention a donc plus particulièrement pour objet une méthode de détermination d'une prédisposition à une maladie thromboembolique veineuse chez un sujet comprenant les étapes consistant à :
i) doser le taux d'anticorps anti-chlamydia dans un échantillon biologique d'un sujet à tester ;
ii) comparer ce taux d'anticorps avec le taux d'anticorps anti-chlamydia obtenu chez des sujets contrôles ;
iii) identifier le sujet testé comme un sujet présentant une prédisposition à une maladie thromboembolique veineuse si le taux d'anticorps obtenu à l'étape i) est supérieur au taux d'anticorps anti-chlamydia obtenu chez des sujets contrôles.

La présente invention porte également sur l'utilisation d'au moins un agent actif contre une infection par une bactérie du genre Chlamydia, en particulier *Chlamydia pneumoniae*, ou d'au moins un agent efficace contre les effets inflammatoires d'une infection par *Chlamydia,* pour la prévention et/ou le traitement d'une maladie thromboembolique veineuse.

De manière préférentielle, des compositions pharmaceutiques contenant des agents antibiotiques peuvent être utilisées.

L'invention a donc plus particulièrement pour objet l'utilisation d'une substance antibiotique active sur les bactéries du genre Chlamydia, notamment *Chlamydia pneumoniae*, pour la préparation d'un médicament destiné à la prévention et/ou au traitement d'une maladie thromboembolique veineuse.

Parmi les substances ou agents antibiotiques actifs sur le genre bactérien Chlamydia, on peut notamment citer les macrolides (par exemple, l'érythromycine ou l'azithromycine), les tétracyclines, les fluoroquinolones et la rifampicine.

Selon un autre mode de réalisation de la présente invention, ledit agent actif contre une infection par *Chlamydia* peut être une protéine immunogène ou un fragment immunogène d'une protéine de *Chlamydia*, notamment de *Chlamydia pneumoniae.* Ces protéines ou ces fragments de protéines sont caractérisés par leur capacité à induire une immunité à médiation cellulaire ou humorale envers une infection par *Chlamydia*, notamment *Chlamydia pneumoniae*, par administration de la protéine en combinaison avec un adjuvant approprié. De manière préférentielle, on peut utiliser la protéine majeure de la membrane externe ou des protéines de la surface cellulaire de la bactérie qui peuvent être fragmentées de manière aléatoire. Les fragments aléatoires d'une protéine de *Chlamydia* peuvent être testés pour leur immunogénicité par l'homme du métier. Des vaccins contenant des antigènes cellulaires de Chlamydia des fragments de celui-ci peuvent être obtenus de manière conventionnelle, par exemple par lyse cellulaire ou par des techniques de purification ou de séparation standard.

Une composition utile selon l'invention peut également comprendre une bacténe *Chlamydia* tuée ou inactivée par tout moyen conventionnel comme la chaleur ou une irradiation.

Une composition utile selon la présente invention peut également contenir une ou des séquences d'acide nucléique codant pour une protéine de surface de *Chlamydia* ou un fragment de celle-ci. L'acide nucléique utilisé peut être administré à l'aide d'un vecteur vaccinal ou sous forme nue, c'est-à-dire exempt de tout agent facilitant la pénétration de cet acide nucléique dans la cellule.

L'invention a également pour objet une méthode pour la prévention et/ou le traitement de la maladie thromboembolique veineuse, dans laquelle on administre à un patient nécessitant un tel traitement une quantité prophylactiquement ou thérapeutiquement efficace d'un agent actif sur les bactéries du genre Chlamydia, notamment d'un agent antibiotique, en association avec un véhicule pharmacéutiquement acceptable.

La prévention des récidives suite à une première thrombose veineuse est plus particulièrement visée.

Les modes d'administration, les posologies et les formes galéniques des compositions pharmaceutiques utiles selon l'invention peuvent être déterminés de manière usuelle par l'homme du métier, notamment selon les critères généralement pris en compte pour l'établissement d'un traitement thérapeutique adapté à un patient, comme par exemple l'âge ou le poids corporel du patient, la gravité de son état général, la tolérance au traitement, et les effets secondaires constatés, etc.

Une composition pharmaceutique utile selon l'invention peut être notamment administrée par voie orale, parentérale, intraveineuse, intramusculaire, sous-cutanée, percutanée ou intra-nasàle.

Lorsque la composition pharmaceutique utilisée est une composition antibiotique, la dose efficace se situe dans les gammes utilisées de manière usuelle, pour tout antibiotique contre les bactéries Chlamydia. On peut notamment administrer de manière avantageuse ladite composition en courts cycles (4 à 10 jours environ) à répéter par exemple tous les six mois après le premier épisode thrombotique veineux.

Le lien entre une infection par une bactérie *Chlamydia pneumoniae* et une maladie thromboembolique veineuse est illustré dans les résultats présentés ci-après, qui ne limitent en aucune manière la portée de la présente invention.

Les auteurs de la présente invention ont ainsi étudié 176 patients avec une maladie thromboembolique veineuse diagnostiquée, et 197 sujets contrôle en bonne santé, d'âge et de sexe variés. Les facteurs de risques acquis pour une maladie thromboembolique veineuse et les facteurs génétiques de prédisposition fréquents (mutations Arg 506 Gln dans le facteur V et G 20210 A dans le facteur II) ont été évalués chez tous les sujets. Le taux d'anticorps IgG anti-*C*. *pneumoniae* a été déterminé par microimmunofluorescence. Tous les échantillons plasmatiques positifs (titre ≥ 128) ont été précisément quantifiés et testés pour la présence d'anticorps IgM spécifiques.

### MATERIELS ET METHODES :

### Sujets :

Parmi 205 patients (92 hommes et 113 femmes) avec des maladies thromboemboliques veineuses, on a sélectionné les patients de moins de 61 ans qui avaient eu au moins un épisode de thrombose veineuse profonde diagnostiquée objectivement (ultrasonographie par compression ou veinographie) et/ou une embolie pulmonaire (scintigraphie pulmonaire de perfusion et de ventilation, angiographie pulmonaire conventionnelle et scanner spiralé). On a réalisé auprès de tous les patients une étude clinique complète soulignant leurs antécédents personnels et familiaux en matière de maladie thromboembolique, et les facteurs de risques acquis (acte chirurgical ou traumatisme dans les trois derniers mois, immobilisation supérieure à 72 heures, grossesse, traitement avec des oestrogènes, veine variqueuse et cancer). On a recueilli le sang de 176 patients (86 %) ; dans 87 cas, les échantillons ont été obtenus moins de trois mois après le déclenchement d'une maladie thromboembolique veineuse aiguë (médiane 1 jour ; écart IQR 0-16), alors que les 89 échantillons restants ont été obtenus plus de trois mois après le déclenchement (médiane 12 mois, écart IQR 6,5-36). Les 197 sujets contrôles sains d'âge et de sexe variés ont été retenus comme n'ayant aucun antécédent de maladie thromboembolique veineuse, d'infarctus du myocarde, de maladie vasculaire périphérique.

### Dosage :

Le sang veineux a été recueilli dans du citrate de trisodium 0,129 M (1:10) et deux étapes de centrifugation à 2 000 g pendant 15 minutes ont été réalisées pour obtenir un plasma pauvre en plaquettes. Le plasma a été congelé et stocké sous forme de fraction aliquote à -40°C jusqu'à utilisation. L'ADN a été extrait à partir des leucocytes par des méthodes standard et stockées à 4°C.

Pour les tests sérologiques, chaque cas et chaque échantillon contrôle ont été marqués avec un nombre aléatoire et analysés en aveugle. Le statut sérologique en *C. pneumoniae* a été déterminé par un dosage par microimmunofluorescence (MIF) à l'aide du kit "SeroFIA IgG Chlamydia" (Savyon Diagnostics LTD, Ashdod, Israël). En bref, des corps élémentaires de *C*. *pneumoniae* purifié (souche IOL 207) ont été utilisés pour détecter les anticorps IgG. Tous les échantillons de plasma étaient criblés initialement à une dilution de 1:128 et ont été considérés positifs au-dessus de cette dilution. Les plasmas positifs ont ensuite été testés à des dilutions de 1:256, 1:512 et 1:1024. Les titres d'IgG spécifiques ont été rapportés comme l'inverse de la dernière dilution positive. Des échantillons positifs pour les IgG *anti-C*. *pneumoniae* ont ensuite été testés pour la présence d'IgM anti-*C*. *pneumoniae* au moyen du dosage MIF avec le kit "SeroFIA IgM Chlamydia" (Savyon Diagnostics LTD), à une dilution de 1:20 comme recommandé par le fabricant.

On a par ailleurs étudié si l'ADN des sujets contrôles et des patients présentaient la mutation Arg 506 Gln dans le facteur V, après amplification par PCR de l'exon 10 du facteur V et digestion par des enzymes de restriction. La transition G 20210 A du gène de la prothrombine a été identifiée après amplification à l'aide de deux amorces :
(5'-TTACAAGCCTGATGAAGGGA-3' et 5'-CCATGAATAGCACTGGGAGCATTGAAGC-3'). La seconde amorce a été construite de telle sorte qu'une substitution nucléotidique (C vers A) à la position 20210 créent un nouveau site de restriction pour *Hind III* lorsque la transition de G vers A à la position 20210 est présente.

### Analyse statistique :

Les données sont analysées en utilisant le logiciel de statistique SAS (Institute Inc., Cary, N.C.). Les caractéristiques cliniques de la population entière des patients et du sous-groupe des cas avec des échantillons de sang prélevés moins de trois mois après l'épisode de thrombose ont été comparées à celles des sujets contrôles en utilisant un test Chi² avec un degré de liberté. L'âge a été testé en analyse de variance (ANOVA).

Les odds ratios associés à une séropositivité pour *C*. *pneumoniae* ont été calculés en comparant les sujets ayant des titres de 256 ou plus ayant des sujets avec des titres inférieurs à 256. L'hétérogénéité des odds ratios en termes d'âge et de sexe a été testée en entrant les variables d'interaction (un degré de liberté) dans les régressions logistiques. Les odds ratios pour une maladie thromboembolique veineuse et l'intervalle de confiance (IC) de 95 % associé à chaque titre d'IgG anti-*C*. *pneumoniae,* codés comme variables binaires, ont été ensuite calculés avec référence à la séronégativité en utilisant une procédure de régression logistique (SAS-PROC LOGIST). La même analyse a été appliquée au sous-groupe de cas avec des échantillons de sang prélevés moins de trois mois après le déclenchement de la maladie thromboembolique veineuse. Dans la population testée entière, les taux de séropositivité pour *C*. *pneumoniae* ont été comparés en terme de première thrombose contre thrombose récurrente, en terme de présence de facteur de risque associé contre absence de facteur de risque associé, et première thrombose à un âge inférieur à 40 ans (âge médian de la population) contre une première thrombose à un âge supérieur à 40 ans, par un test Chi² avec un degré de liberté. Les différences avec des valeurs p inférieures à 0,05 ont été considérées comme significatives.

### RESULTATS :

Les cas et contrôles ne différent pas en termes de ratios d'âge ou de sexe (cf tableau 1). La maladie thromboembolique veineuse a été considérée comme idiopathique chez des patients n'ayant pas de contraception orale, de chirurgie récente (inférieure à un mois) ou de traumatisme, de grossesse ou d'accouchement, d'immobilisation ou de cancer. La moitié des patients a été recrutée dans les trois mois après l'épisode aigu de thrombose veineuse. Les caractéristiques de ce sous-groupe n'étaient pas statistiquement différentes de celles de la population entière de cas. La prévalence des mutations Arg 506 Gln du facteur V et G 20210 A du facteur II était dans le domaine attendu chez des Caucasiens (21,9 % chez les patients et 5,1 % chez les contrôles, p<0,0001 ; et 10,2 % et 4,1 %, p=0,02, respectivement).

Les titres IgG spécifiques de *C*. *pneumoniae* tendent à être plus élevés chez les patients que chez les contrôles (cf tableau 2). De manière significative, on observe que plus de patients que de contrôles ont des titres IgG de *C*. *pneumoniae* de 256 ou plus (54 % et 15,9 % respectivement, p< 0,0001). L'odds ratio pour les maladies thromboemboliques veineuse associées avec des titres IgG de 256 ou plus était de 6,2 (Intervalle de confiance (IC) 95 %, 3,8-10,1). Dans le sous-groupe de patients avec des échantillons de sang prélevés moins de trois mois après l'épisode thrombotique, le ratio brut (odds ratio) parmi les sujets avec des titres IgG≥ 256 était de 5,4 (IC 95 %, 3,1-9,6). De plus, l'odds ratio pour une maladie thromboembolique veineuse augmente avec le titre d'IgG : pour des titres de 256, 512 et 1024, les odds ratio bruts étaient de 2,1 (IC 95 %, 1,0-4,2), 4,3 (2,1-8,9) et 32,4 (4.2-248.3), respectivement (cf tableau 3). Une plus large proportion de contrôles séropositifs avait un titre IgG bas de 128 par rapport aux témoins (54,9 % et 21 %, respectivement). Des odds ratios similaires ont été obtenus dans le sous-groupe de cas testés dans les trois mois après l'épisode thrombotique.

Les odds ratios n'étaient pas différents significativement selon l'âge ou le sexe. Après exclusion des sujets portant les mutations Arg 506 Gln du facteur V et G 20210 A du facteur II, l'odds ratio pour la maladie thrombembolique veineuse associée à un titre supérieur à 256 était de 7,7 (IC 95 %, 4,5-13,2). Les caractéristiques de la maladie thrombembolique veineuse (âge au moment du premier épisode ; nature récurrente ou spontanée) ne différaient pas en fonction du statut sérologique de *C. pneumoniae.*

Pour discriminer une infection aiguë d'une infection chronique, les auteurs de l'invention ont en outre évalué les anticorps IgM anti-*C*. *pneumoniae* circulants dans les 95 cas et 31 contrôles qui avaient des titres d'IgG supérieurs à 256. Seul un sujet du groupe des patients atteints de maladies thromboemboliques veineuse était positif pour les IgM.

### CONCLUSION :

Les résultats ci-dessus montrent clairement un lien entre le statut sérologique de *C. pneumoniae* et la thrombose veineuse. L'odds ratio pour une maladie thromboembolique veineuse associée avec des titres circulants d'anticorps IgG anti-*C*. *pneumoniae* de 256 ou plus était de 6,2 (IC 95 % ; 3,8-10,1) et restait élevé après exclusion des patients avec des mutations Arg 506 Gln dans le facteur V et G 20210 A dans le facteur II (odds ratio 7,7 ; IC 95 % ; 4,5-13,2). Le fait que l'odds ratio pour une maladie thromboembolique veineuse augmente avec le titre d'anticorps IgG renforce cette association (cf tableau 3).

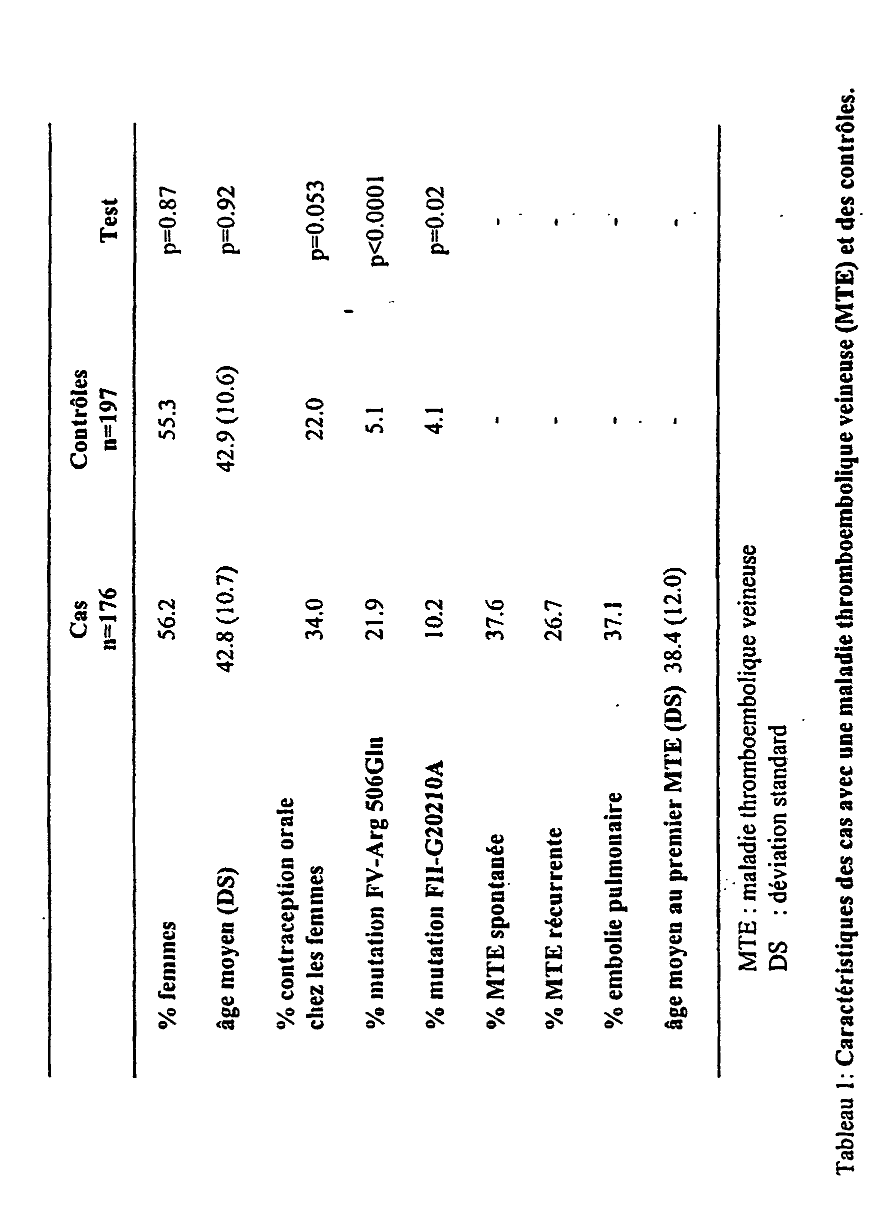

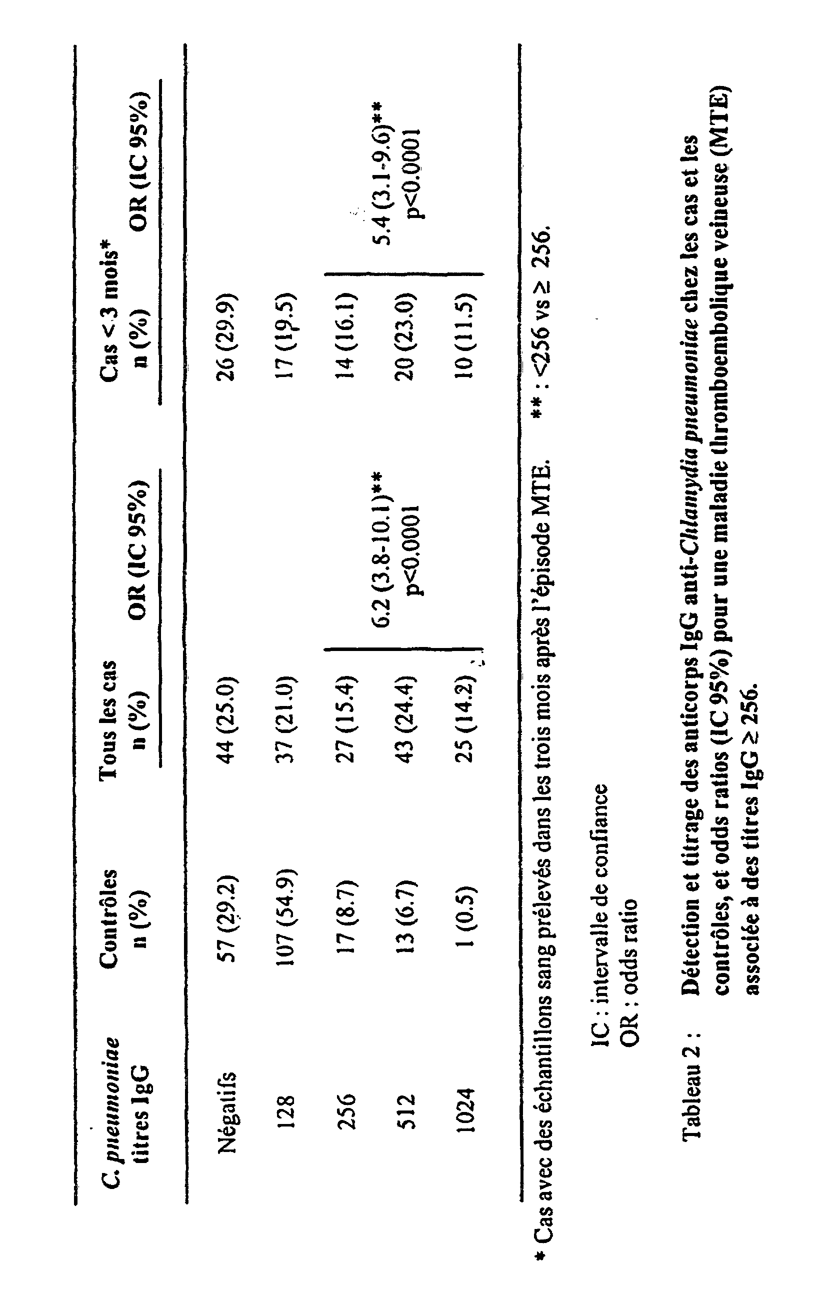

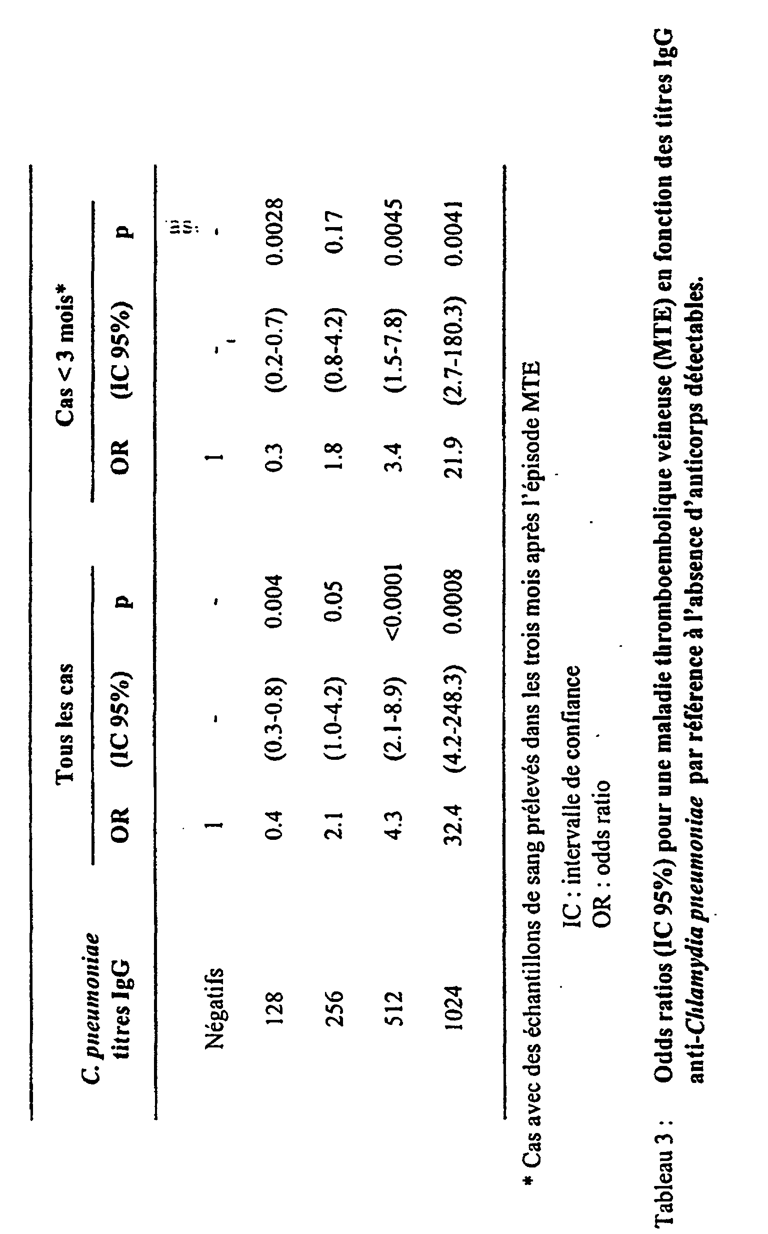

### REFERENCES

- Bartels C. et al. *Detection of Chlamydia pneumoniae but not cytomegalovirus in occluded saphenous vein coronary artery bypass grafts*. Circulation, 1999, 99:879-882.
- Gibbs RGJ, Carey N, Davies AH. *Chlamydia pneumoniae and vascular disease*. Br J Surg 1998;85:1191-7.
- Ong G, Thomas BJ, Mansfield AO, Davidson BR, Taylor-Robinson D. *Detection and widespread distribution of Chlamydia pneumoniae in the vascular system and its possible implications.* J Clin Pathol 1996;49:102-6.
- Rosendaal FR. *Thrombosis in the young : epidemiology and risk factors*. *A focus on venous thrombosis.* Thromb Haemost 1997;78:1-6.

## Revendications

1. Méthode de détermination *in vitro* d'une prédisposition à une maladie thromboembolique veineuse chez un sujet, dans laquelle on détermine si le sujet a été infecté par une bactérie du genre Chlamydia; notamment *Chlamydia pneumoniae.*

2. Méthode selon la revendication 1, dans laquelle on détermine si le sujet a été infecté par une bactérie du genre Chlamydia, notamment *Chlamydia pneumoniae,* par dosage du taux d'anticorps anti-Chlamydia dans un échantillon biologique dudit sujet.

3. Utilisation d'au moins un agent actif contre une infection par une bactérie du genre Chlamydia, en particulier *Chlamydia pneumoniae*, ou d'au moins un agent efficace contre les effets inflammatoires d'une infection par Chlamydia, pour la préparation d'un médicament destiné à la prévention et/ou au traitement d'une maladie thromboembolique veineuse.

4. Utilisation selon la revendication 3, dans laquelle ledit agent actif contre une infection par une bactérie du genre Chlamydia, en particulier *Chlamydia pneumoniae*, est une substance antibiotique.

5. Utilisation selon la revendication 4, dans laquelle ladite substance antibiotique active sur les bactéries du genre Chlamydia, en particulier *Chlamydia pneumoniae,* est choisie parmi le groupe constitué par les macrolides, les tétracyclines, les fluoroquinolones et la rifampicine.

6. Utilisation selon l'une quelconque des revendications 3 à 5, dans laquelle ledit médicament est destiné à la prévention des récidives suite à une première thrombose veineuse.

## Patentansprüche

1. Verfahren zum *in-vitro*-Nachweis einer Prädisposition für eine venöse thramboembolische Erkrankung eines Menschen, in welchem ermittelt wird, ob dieser mit einer Bakterie der Gattung Chlamydia, insbesondere *Chlamydia pneumoniae*, infiziert gewesen ist.

2. Verfahren nach Anspruch 1, in welchem ermittelt wird, ob ein Mensch mit einer Bakterie der Gattung Chlamydia, insbesondere *Chlamydia pneumoniae*, infiziert gewesen ist, indem der Gehalt an Anti-Chlamydia-Antikörpern in einer biologischen Probe dieses Menschen gemessen wird.

3. Verwendung mindestens eines Wirkstoffs gegen eine Infektion mit einer Bakterie der Gattung Chlamydia, insbesondere *Chlamydia pneumoniae*, oder mindestens eines Wirkstoffs gegen die entzündlichen Folgen einer Chlamydien-Infektion zur Herstellung eines Arzneimittels, das zur Verhütung und/oder Behandlung einer venösen thromboembolischen Erkrankung vorgesehen ist.

4. Verwendung nach Anspruch 3, bei welcher der Wirkstoff gegen eine Infektion mit einer Bakterie der Gattung Chlamydia, insbesondere *Chlamydia pneumoniae*, ein Antibiotikum ist.

5. Verwendung nach Anspruch 4, bei welcher das Antibiotikum, das gegen die Bakterien der Gattung Chlamydia, insbesondere *Chlamydia pneumoniae*, wirkt, aus der Gruppe ausgewählt wird, die aus Makroliden, Tetracyclinen, Fluorchinolonen und Rifampicin besteht.

6. Verwendung nach einem der Ansprüche 3 bis 5, bei welcher das Arzneimittel zur Verhütung von Rezidiven nach einer ersten Venenthrombose vorgesehen ist.

## Claims

1. Method for the *in vitro* detection of a predisposition to venous thromboembolic disease in a subject, in which it is determined whether the subject has been infected with a bacterium of the genus *Chlamydia*, especially *Chlamydia pneumoniae*.

2. Method according to claim 1, in which it is determined whether the subject has been infected by a bacterium of the genus *Chlamydia*, especially *Chlamydia pneumoniae*, by analysing the level of anti-*Chlamydia* antibodies in a biological sample from said subject.

3. Use of at least one agent that is active against an infection by a bacterium of the genus *Chlamydia*, especially *Chlamydia pneumoniae*, or of at least one agent that is effective against the inflammatory effects of a *Chlamydia* infection, in the preparation of a medicament for the prevention and/or treatment of venous thromboembolic disease.

4. Use according to claim 3, in which said agent that is active against an infection by a bacterium of the genus *Chlamydia,* especially *Chlamydia pneumoniae*, is an antibiotic substance.

5. Use according to claim 4, in which said antibiotic substance that is active against bacteria of the genus *Chlamydia,* especially *Chlamydia pneumoniae*, is selected from the group consisting of macrolides, tetracyclines, fluoroquinolones and rifampicin.

6. Use according to any one of claims 3 to 5, in which said medicament is intended for the prevention of recurrences following a first venous thrombosis.
